# EUROPEAN PATENT APPLICATION

(11) **EP 4 644 366 A1**
(43) Date of publication of application: **05.11.2025**
(21) Application number: 23910212.2
(22) Date of filing: 15.12.2023
(51) Int. Cl.: C07C 309/73, C07C 67/31, C07D 405/06, C07C 303/28, C07C 309/66, C07C 67/08, C07C 69/675, C07C 67/343, C07C 67/30, C07C 69/63

(54) **PREPARATION METHODS FOR PILOCARPINE AND INTERMEDIATE COMPOUND THEREOF**

(30) Priority: 29.12.2022 CN 202211712042
(71) Applicant: Zhejiang Ausun Pharmaceutical Co., Ltd., Taizhou, Zhejiang 317016 (CN)
(72) Inventor: DAI, Chunguang, Linhai Taizhou, Zhejiang 317016 (CN); GUO, Jiajia, Linhai Taizhou, Zhejiang 317016 (CN); ZHANG, Lirong, Linhai Taizhou, Zhejiang 317016 (CN); YU, Guanneng, Linhai Taizhou, Zhejiang 317016 (CN); ZHANG, Shengkang, Linhai Taizhou, Zhejiang 317016 (CN); ZHENG, Zhiguo, Linhai Taizhou, Zhejiang 317016 (CN)
(74) Representative: Bianchetti & Minoja with Trevisan & Cuonzo IPS SRL
(86) International application number: PCT/CN2023/139150
(87) International publication number: WO 2024/140282

(57) **Abstract**

The invention relates to a process for preparing pilocarpine and intermediate compounds therefor. In particular, the present invention relates to key intermediates for the preparation of pilocarpine, i.e., compounds of formula I and formula II, and processes for their preparation. One method is to use malic acid as a raw material, react it with alcohol or sulfate ester first, then react with haloethane, etc. to obtain the compound of formula II, and finally react with a halogenating reagent to obtain the compound of formula I; in the other method, n-butyraldehyde is used as a raw material, and is subjected to condensation reaction, oxidation reaction, esterification reaction and sulfonylation reaction in sequence to obtain the compound of formula II, and finally react with a halogenating reagent to obtain the compound of formula I. The preparation method of the present invention has the advantages of mild reaction conditions, simple reaction process, high overall yield and high purity of the target product, and is therefore very suitable for industrial production.

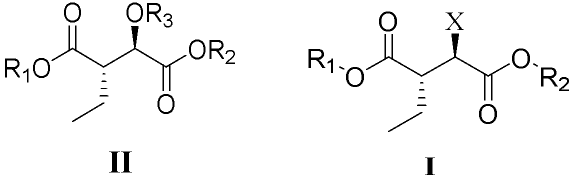

## Description

### Technical Field

The present invention relates to the field of medicinal chemistry, and more particularly to compounds that are intermediates in the synthesis of pilocarpine and to processes for preparing these intermediate compounds.

### Background

Glaucoma is an eye disease in which the intraocular pressure is intermittently or continuously elevated. Persistent ocular hypertension can cause damage to various parts of the eyeball and visual function, and the visual field can be completely lost and even blindness can occur if not treated in time.

Pilocarpine is an alkaloid which mimics the action of acetylcholine and is extracted from the leaves of *Pilocarpus Brasiliflorus* and *Pilocarpus microphyllus Stapf.*

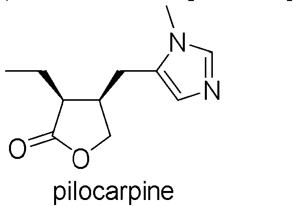

Pilocarpine can be used for the treatment of primary glaucoma, including open angle and closed angle glaucoma. Compared with physostigmine, pilocarpine has a mild and short-lived effect, and its aqueous solution is more stable. Pilocarpine can also be used for salivary gland hypofunction, and its oral tablet SALAGEN can relieve xerostomia. In addition, pilocarpine can also be used for miosis in cataract intraocular lens implantation surgery and symptomatic treatment of atropine drug poisoning. At present, medicinal pilocarpine is isolated and extracted from plants. However, as the requirements for environmental protection continue to increase, extraction from plants becomes more and more difficult. Therefore, if pilocarpine can be obtained by chemical synthesis, higher social benefit and economic benefit can be obtained.

The chemical synthesis of pilocarpine has always been a hot research topic, and early literature Tetrahedron, 1972, 28, 967-972 and patents JP03161481 and US 5182198 reported its synthesis route. This route uses furfural as raw material and uses enzymes to resolve homopilopic acid ester. However, homopilopic acid ester is prone to isomerization when hydrolyzing 2-ethyl group under alkaline conditions, and it is necessary to undergo an enzymatic reaction again to obtain (+)-homopic acid. This method has a long route and uses dangerous materials sodium metal and noble metal rhodium. In addition, this route uses expensive enzymatic hydrolysis resolution, and the resolution yield is low. All these bring great inconvenience to industrial production.

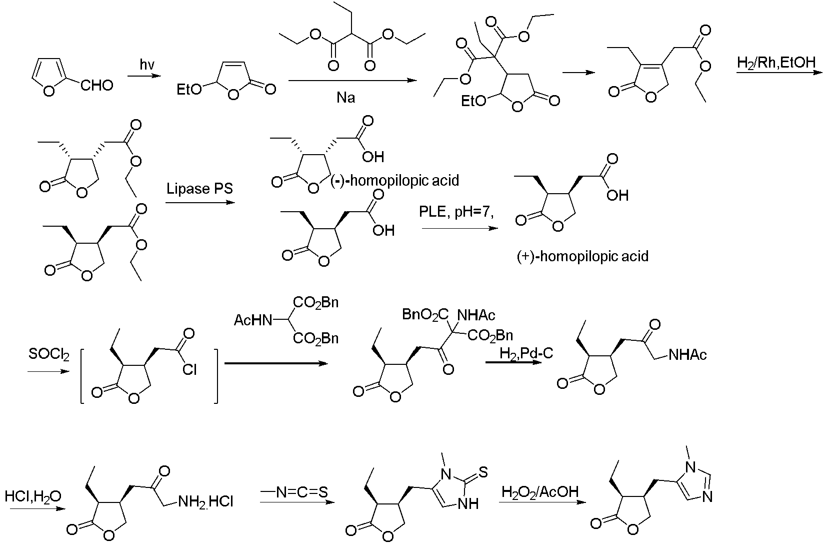

In view of the problem that the chemical properties of pilocarpine free base are unstable and the 2-ethyl group is easily isomerized into thermodynamically stable isopilocarpine under alkaline conditions, 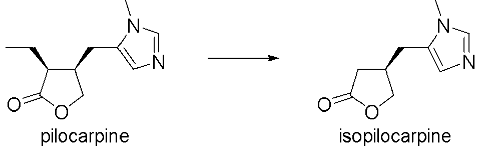 Helvetica Chimica Acta, 1972, vol.55, p.1053-1062 attempted to synthesize racemic pilosinine by steps such as Stobbe condensation of diethyl succinate, and then the desired (+)-pilosinine was obtained through chiral resolution. In order to stereoselectively introduce cis-2-ethyl, it is necessary to undergo 2-acetylation, hydrogenation reduction, dehydration, and hydrogenation to obtain a 75/25 mixture of pilocarpine and isopilocarpine, and then recrystallization purification by salt formation to obtain pure pilocarpine. The resolution efficiency of the intermediate pilosinine in this method is extremely low; moreover, the introduction of 2-ethyl group requires the use of metal platinum, and the stereoselectivity is not high, making the subsequent separation and purification very difficult.

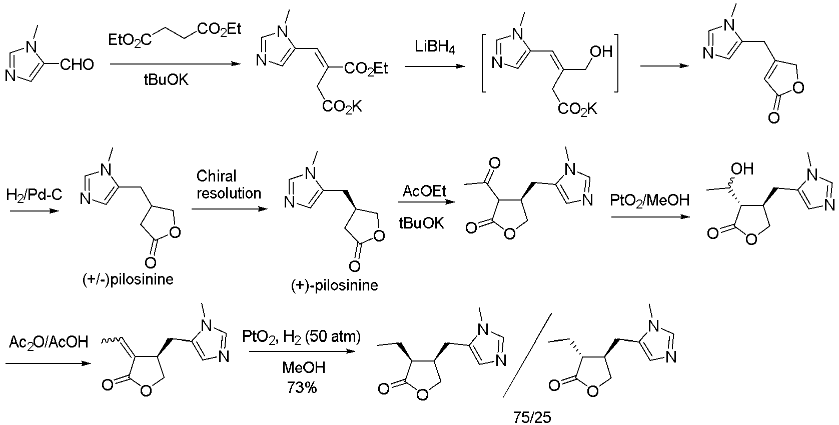

The subsequent literature, Tetrahedron Letters, Vol. 33, No. 18, 2441-2450, 1992, attempted to improve the synthesis of the intermediate pilosinine by using Even's asymmetric alkylation. However, the yield of the asymmetric alkylation step in this method is only 50%, and the reaction period of reductive removal of auxiliary groups is too long, making it difficult to industrialize.

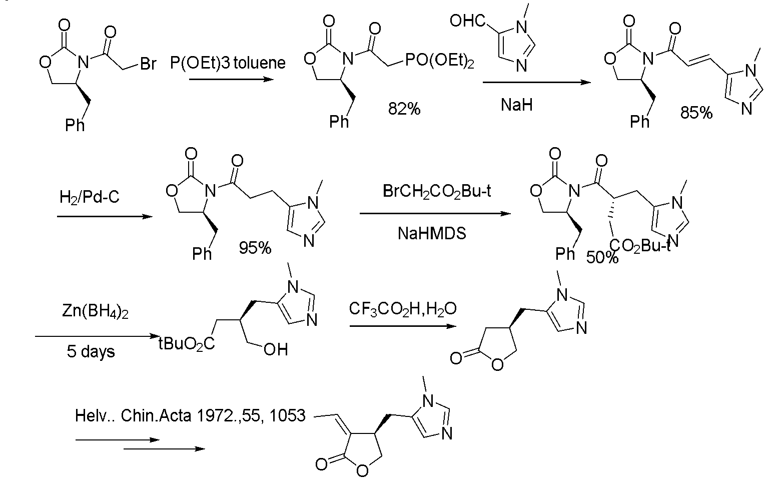

The document J. AM. CHEM. SOC. 2002, 124, 8198-8199 reports that under the catalysis of metal rhodium and chiral phosphine ligand, (4R)-(Z)-dehydrohomopilopic aldehyde can be obtained enantioselectively and efficiently from butynic acid cis-butenediol monoester, which is then subjected to catalytic hydrogenation to give homopilopic aldehyde, the latter undergoes [3+2] cycloaddition and elimination reaction with *p*-methylsulfonyl methyl isonitrile and methylamine to obtain pilocarpine. However, the method needs to use 5% noble metal rhodium, the stereoselectivity of hydrogenation to obtain homopilopic aldehyde is poor, and the reaction period for introducing the diazole heterocycle in the last step is too long, making said method unsuitable for industrial production.

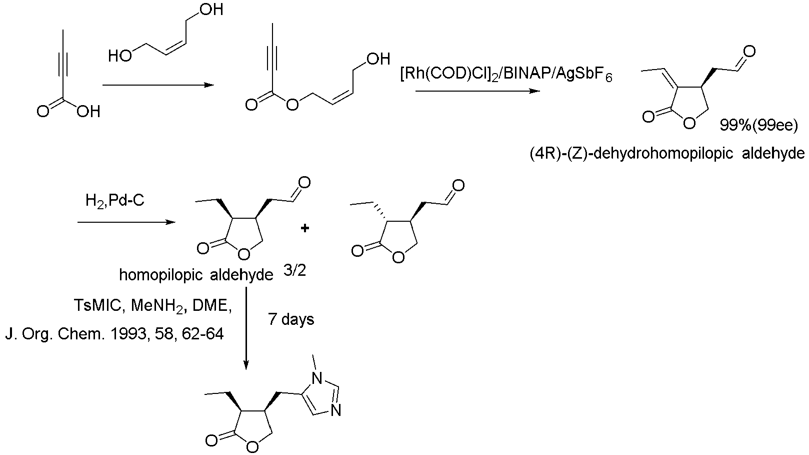

Tetrahedron 65, 2009, 8283-8296 reported a process using diethyl malonate as raw material, alkylating it with 2-bromo-1,1-dimethoxyethane in the presence of sodium ethoxide and reducing with aluminum hydride Lithium to give the diol intermediate, which is then enzymatically esterified to yield the chiral mono-alcohol. Dess-Martin oxidation yields the corresponding aldehyde, which undergoes an addition reaction with vinyl magnesium bromide to obtain a chiral diol, cyclizing with carbonyldiimidazole to obtain 1,3-dioxane-2-one intermediate, and reacting the intermediate in the presence of metal palladium and carbon monoxide to obtain chiral acetal lactone. Then carrying out deprotection, [3+2] cycloaddition and elimination reaction with *p*-methylsulfonyl methyl isonitrile and methylamine, catalytic hydrogenation and the like to obtain pilocarpine nitrate. The reaction steps of this route are long, the intermediates are all oily substances, the purification in each step is difficult, biological enzyme and two-step noble metal palladium and platinum catalytic reactions are involved, and the cost is high.

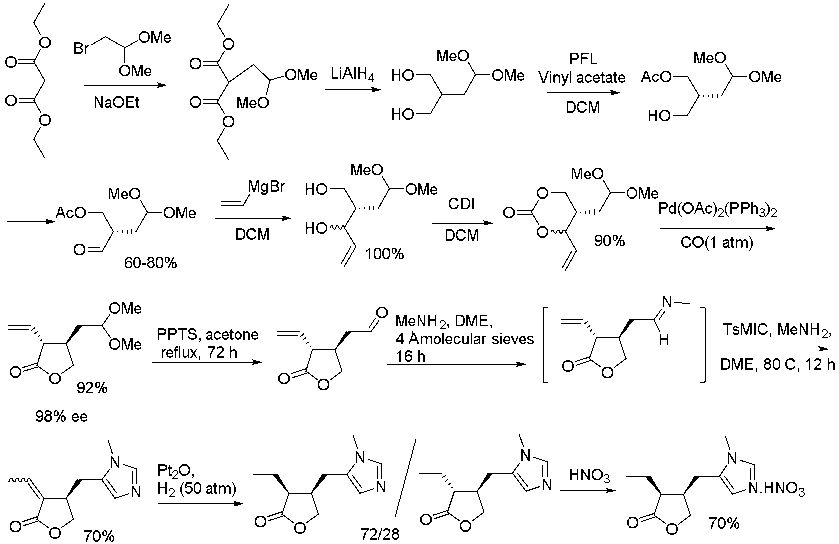

The Journal of Organic Chemistry, 1993, vol. 58, # 5, p. 1159-1166 and WO9221675 reported a process for the preparation of pilocarpine hydrochloride starting from L-aspartic acid by stereoselective alkylation of amino groups protected with bulky protecting reagents (9-bromo-9-phenylfluorene (PhFIBr)), deprotection, diazotization bromination, esterification and then by Zn(Ag)-mediated Reformatsky reaction to give a trisubstituted lactone intermediate with a ratio of alpha configuration/beta configuration of methyl formate at the 4-position of the lactone ring of about 9/1. Then, the pilocarpine is finally obtained through hydrogenolysis, selective reduction of carboxyl by LiBH4 and lactonization reaction. However, the reaction steps for preparing the compound (2S,3R)-2-bromo-3-ethylsuccinic acid dimethyl ester of the formula I are long, expensive 9-bromo-9-phenylfluorene (PhFIBr) reagent is needed for protecting the amino group of the raw material aspartic acid, and the introduction of the protecting group needs the environmentally unfriendly reagent Pb(NO₃)₂; in addition, the protecting group has a large molecular weight and is poor in atom economy; moreover, the Reformatsky reaction for introducing imidazole heterocycle needs to use Zn (Ag) alloy, and the preparation process of Zn(Ag) is cumbersome and poses safety risks in industrial production. These shortcomings severely limit the industrial production of this route.

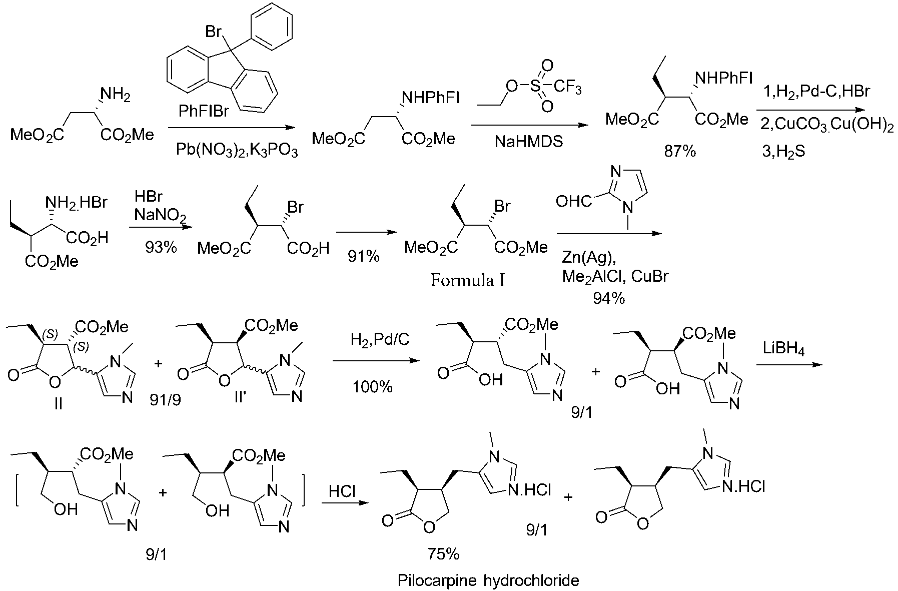

Therefore, how to prepare the above-mentioned compound of formula I simply and efficiently has become the key to solving the problem of industrial production of pilocarpine.

### Disclosure of Invention

The present invention overcomes the disadvantages of the processes of the prior art and provides intermediates of formula II and formula I for the preparation of pilocarpine and processes for their preparation. The present invention also provides processes for the preparation of pilocarpine using said intermediates. The preparation method of the present invention has the advantages of mild reaction conditions, simple reaction process, high overall yield and high purity of the target product, and is therefore very suitable for industrial production.

### Definition

For the purpose of interpreting this specification, the following definitions will be used, and where appropriate, terms used in the singular may also include the plural, and vice versa. It is to be understood that the terminology used herein is for the purpose of describing embodiments only, and is not intended to be limiting.

The term "halogen" or "halo" as used herein refers to F, Cl, Br or I. Furthermore, the term "halogen-substituted" group is intended to include mono- or polyhalogenated groups wherein one or more hydrogens in the group are replaced by one or more halogens, which may be the same or different.

The term "alkyl" as used herein refers to a straight or branched chain saturated hydrocarbon group consisting of carbon atoms and hydrogen atoms. In particular, the alkyl group has 1-10, e.g., 1 to 8, 1 to 6, 1 to 5, 1 to 4, 1 to 3, or 1 to 2 carbon atoms. For example, as used herein, the term "C₁₋₈alkyl" refers to a straight or branched chain saturated hydrocarbon group having 1 to 8 carbon atoms, examples of which are methyl, ethyl, propyl (including n-propyl and isopropyl), butyl (including n-butyl, isobutyl, sec-butyl or tert-butyl), pentyl (including n-pentyl, isopentyl, neopentyl), n-hexyl, 2-methylpentyl and the like.

The term "haloC₁₋₈alkyl" as used herein refers to a C₁₋₈alkyl group as described above, in which one or more (e.g., 1, 2, 3, 4, 5 or 6) hydrogen atoms are replaced by halogen. It will be understood by those skilled in the art that when there is more than one halogen substituent, the halogens may be the same or different and may be located on the same or different carbon atoms. Examples of "haloC₁₋₈alkyl" include -CH₂F, -CHF₂, -CF₃, -CCl₃, -C₂F₅, -C₂Cl₅, -CH₂CF₃, -CH₂Cl, -CH₂CH₂CF₃ or -CF(CF₃)₂, and the like.

The term "alkoxy", alone or in combination with other groups, refers to the group R^{y}-O-, wherein R^{y} is alkyl as described above. For example, "C₁₋₈alkoxy" represents the group R^{y}-O-, wherein R^{y} is a C₁₋₈alkyl as described above.

"C₃₋₈cycloalkyl" refers to a cyclic saturated hydrocarbon group containing 3 to 8 ring carbon atoms, examples of which include but are not limited to: cyclopropyl, cyclobutyl, cyclopentyl, cyclohexyl, cycloheptyl, cyclooctyl, methylcyclobutyl, and the like.

"Aryl" refers to a monocyclic or fused bicyclic aromatic ring consisting of carbon and hydrogen atoms. "C₆₋₁₀aryl" refers to an aryl group containing 6 to 10 carbon atoms. For example, aryl may be phenyl or naphthyl.

"Aralkyl" refers to an alkyl group as described above substituted with an aryl group as described above, preferably C₆₋₁₀aryl-C₁₋₈alkyl, such as benzyl.

"Aralkoxy" refers to an alkoxy group as described above substituted with an aryl group as described above, such as benzyloxy.

"Acyl" refers to the group -CO-Rₓ, wherein Rₓ is alkyl, cycloalkyl, aryl or aralkyl as described above, such as alkanoyl or aralkanoyl, for example acetyl or benzoyl.

"Sulfonyl" refers to the group -S(O)₂-Rₓ, wherein Rₓ is alkyl, cycloalkyl, aryl or aralkyl as described above, such as methanesulfonyl, ethanesulfonyl, benzenesulfonyl, *p*-toluenesulfonyl, *p*-chlorobenzenesulfonyl, *p*-nitrobenzenesulfonyl or *m*-dinitrobenzenesulfonyl.

The aryl groups mentioned above, either as the aryl group itself or as part of other groups such as aralkyl, aralkoxy, acyl, or sulfonyl, may be optionally substituted with one or more substituents. When the aryl group is substituted, said substituent is selected from C₁₋₈alkyl, C₁₋₈alkoxy, haloC₁₋₈alkyl, haloC₁₋₈alkoxy, halogen, hydroxy and nitro, more preferably methyl, ethyl, methoxy, ethoxy, halogen or nitro. For example, a substituted benzenesulfonyl group can be a benzenesulfonyl group having one or more substituents selected from C₁₋₈alkyl, haloC₁₋₈alkyl, halogen, or nitro on the benzene ring.

In a first aspect, the present invention provides an intermediate compound of formula II for the synthesis of pilocarpine: 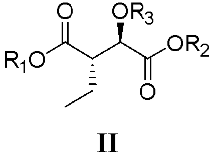
wherein R₃ is -S(O)₂-Rₓ and Rₓ is C₁₋₈alkyl, C₃₋₈cycloalkyl, C₆₋₁₀aryl, or C₆₋₁₀aryl-C₁₋₈alkyl, each of which is optionally substituted with one or more substituents selected from the group consisting of C₁₋₈alkyl, C₁₋₈alkoxy, haloC₁₋₈alkyl, halogen and nitro, and
R₁ and R₂ may be the same or different, and are each independently selected from the group consisting of C₁₋₈alkyl, C₃₋₈cycloalkyl, C₆₋₁₀aryl, or C₆₋₁₀aryl-C₁₋₈alkyl, wherein said C₁₋₈alkyl, C₃₋₈cycloalkyl, C₆₋₁₀aryl, or C₆₋₁₀aryl-C₁₋₈alkyl are each optionally substituted with one or more of C₁₋₈alkyl, C₁₋₈alkoxy, haloC₁₋₈alkyl, haloC₁₋₈alkoxy, halogen, hydroxy, and nitro,
preferably, R₁ and R₂ are each independently selected from C₁₋₈alkyl, benzyl, C₁₋₈alkyl substituted benzyl, C₁₋₈alkoxy substituted benzyl or halogen substituted benzyl.

In a preferred embodiment, R₃ is selected from the group consisting of methanesulfonyl, ethanesulfonyl, benzenesulfonyl, *p*-toluenesulfonyl, *p*-chlorobenzenesulfonyl, *p*-nitrobenzenesulfonyl, or *m*-dinitrobenzenesulfonyl.

In a second aspect, the present invention provides a process for the preparation of the compound of formula II, comprising the steps of: 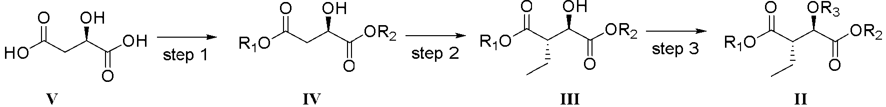
wherein R₁, R₂ and R₃ are as defined above in the first aspect, X is Cl, Br or I,
step 1: reacting a compound of formula V with an alcohol of formula R_{y}-OH or with a sulphate ester to obtain a compound of formula IV, wherein R_{y} is as defined for R₁ and R₂ above in the first aspect;
step 2: reacting the compound of formula IV with a halogenated hydrocarbon CH₃CH₂X under basic conditions to obtain a compound of formula III;
step 3: reacting the compound of formula III with a sulfonylating agent under basic conditions to obtain the compound of formula II.

In one embodiment, the sulfate ester in step 1 is selected from dimethyl sulfate, diethyl sulfate, diisopropyl sulfate, or dipropyl sulfate.

In one embodiment, the basic conditions described in step 2 are achieved by adding one or more reagents selected from LiHMDS, NaHMDS, LDA, n-butyllithium, t-butyllithium, NaH.

In one embodiment, the basic conditions described in step 3 are achieved by adding one or more reagents selected from the group consisting of methylamine, ethylamine, propylamine, cyclopropylamine, n-butylamine, t-butylamine, n-pentylamine, isopentylamine, t-pentylamine, cyclopentylamine, hexylamine, cyclohexylamine, diethylamine, ethylenediamine, diisopropylethylamine, triethylamine, ethanolamine, aniline, phenethylamine, and benzylamine.

In one embodiment, the sulfonylating agent used in step 3 is X-S(O)₂-Rₓ , wherein Rₓ is as defined in claim 1 and X is Cl, Br or I, preferably, said sulfonylating agent is selected from methanesulfonyl chloride, ethanesulfonyl chloride, benzenesulfonyl chloride, *p*-toluenesulfonyl chloride, *p*-chlorobenzenesulfonyl chloride, *p*-nitrobenzenesulfonyl chloride or m-dinitrobenzenesulfonyl chloride, methanesulfonyl bromide, ethanesulfonyl bromide, benzenesulfonyl bromide, *p*-toluenesulfonyl bromide, *p*-chlorobenzenesulfonyl bromide, *p*-nitrobenzenesulfonyl bromide or *m*-dinitrobenzenesulfonyl bromide.

Typically, the compound of formula V is reacted with an equivalent or excess of acid chloride in an organic solvent at an appropriate temperature to obtain the acid chloride of the compound of formula V, which is then reacted with an alcohol or phenol to obtain a compound of formula IV wherein R₁ = R₂. When preparing a monoacyl chloride, the compound of formula IV can be obtained as a monoesterified compound by adding an alcohol or phenol, followed by repeating the acid chlorination again and adding a different alcohol or phenol reagent to obtain a compound of formula IV wherein R₁ ≠ R₂.

The compound of formula IV is dissolved in an organic solvent, 1.0-1.2 equivalents of alkali was added dropwise while controlling the reaction temperature at -80 to -20 °C and, after the dropwise addition is completed, keeping the temperature for 30mins. Then an equivalent amount of halogenated hydrocarbon CH₃CH₂X is added, until the reaction is complete. After conventional quenching, extraction, phase separation, and concentration, the compound of formula III is obtained.

The compound of formula II is prepared by conventional reactions in an organic solvent under basic conditions.

In a third aspect, the present invention provides an alternative process for the preparation of a compound of formula II, comprising the steps of: 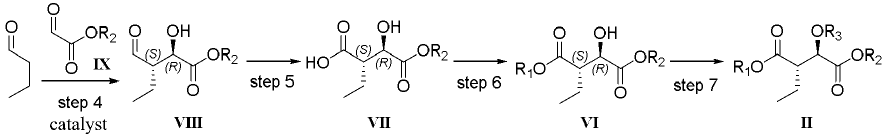
step 4: carrying out condensation reaction of n-butyraldehyde and a compound of formula IX under catalysis to obtain a compound of formula VIII;
step 5: subjecting the compound of formula VIII to oxidation reaction to obtain a compound of formula VII;
step 6: esterifying the compound of formula VII to obtain a compound of formula VI; and
step 7: subjecting the compound of formula VI to sulfonylation reaction to obtain the compound of formula II;
wherein R₁, R₂ and R₃ are as defined above in the first aspect.

In one embodiment, said catalyst of step 4 is: 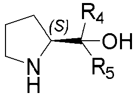 wherein R₄ and R₅ are each independently selected from the group consisting of C₆₋₁₀aryl, C₁₋₈alkyl substituted C₆₋₁₀aryl and halogen substituted C₆₋₁₀aryl, preferably phenyl. In a preferred embodiment, said catalyst is

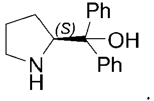

In the above mentioned process, the catalyst used can be prepared according to the method described in the reference "The Journal of Organic Chemistry, 2002, 67, 22, 7769-7773".

In one embodiment, step 4 is an asymmetric aldol condensation reaction of n-butyraldehyde and the compound of formula II carried out in an aqueous organic solvent. The reaction selectively generates the compound of formula VIII, which is reacted in step 5 by direct addition of an oxidizing agent without isolation and purification. Said aqueous organic solvent refers to a mixed solvent of any one or combination of tetrahydrofuran, methyltetrahydrofuran, acetone, acetonitrile, N,N-dimethylformamide, N,N-dimethylacetamide, dimethyl sulfoxide and N-methylpyrrolidone and water in any ratio. The catalyst is used in an amount of 1.0 to 20% equivalent, and the reaction temperature is 0 to 35°C.

In one embodiment, the oxidizing agent used in step 5 is selected from any one of hydrogen peroxide, tert-butyl hydroperoxide, sodium hypochlorite, sodium chlorite, sodium chlorate, or a combination thereof.

In one embodiment, in step 6, the compound of formula VII is esterified with R₁OH under acidic conditions to prepare the compound of formula VI. The acidic condition is sulfuric acid, hydrochloric acid, hydrobromic acid or phosphoric acid. The R₁ is as defined above and is selected from the group consisting of C₁₋₈alkyl, C₃₋₈cycloalkyl, C₆₋₁₀aryl, C₁₋₈alkyl-substituted C₆₋₁₀aryl, benzyl, C₁₋₈alkyl substituted benzyl, C₁₋₈alkoxy substituted benzyl or halogen substituted benzyl; preferably, R₁ is selected from the group consisting of C₁₋₈alkyl, benzyl, C₁₋₈alkyl substituted benzyl, C₁₋₈alkoxy substituted benzyl or halogen substituted benzyl.

In one embodiment, in step 7, the compound of formula VI is reacted with a sulfonylating reagent to produce a compound of formula II, wherein the sulfonylating reagent and the R₃ group are as described above. The reaction solvent is an aprotic organic solvent, including toluene, acetonitrile, isopropyl ether, tetrahydrofuran, 2-methyltetrahydrofuran, methyl tert-butyl ether, dichloromethane, trichloromethane, ethyl acetate, isopropyl acetate, N,N-dimethylformamide, N,N-dimethylacetamide and N-methylpyrrolidone.

In a fourth aspect, the invention provides a process for the preparation of a compound of formula I, 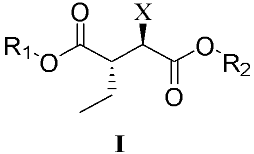 comprising the step of: reacting a compound of formula II with a halogenating agent to give the compound of formula I 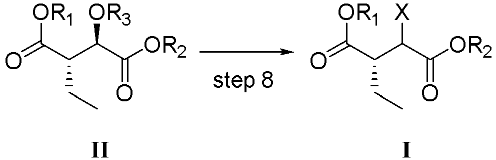 wherein R₁, R₂ and R₃ are as defined above for the first aspect, and X is Cl, Br or I.

In one embodiment, the halogenating agent is selected from the group consisting of chlorine, NCS, trichloroisocyanuric acid, dichlorohydantoin, lithium chloride, sodium chloride, potassium chloride, tetrabutylammonium chloride, bromine, NBS, dibromohydantoin, tribromoisocyanuric acid, lithium bromide, sodium bromide, potassium bromide, tetrabutylammonium bromide, iodine, diiodohydantoin, lithium iodide, sodium iodide, potassium iodide and tetrabutylammonium iodide.

In a fifth aspect, the invention provides a process for the preparation of pilocarpine, which is characterized by comprising the following steps: 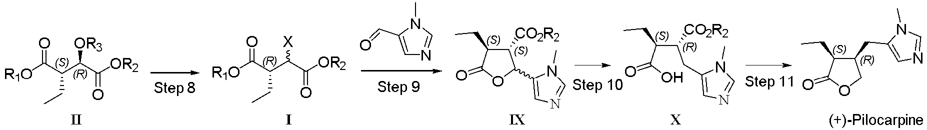
step 8: reacting a compound of formula II with a halogenating agent to obtain a compound of formula I;
step 9: reacting the compound of formula I with 1-methylimidazole-5-carbaldehyde to obtain a compound of formula IX;
step 10: reacting the compound of formula IX under the action of a reducing agent to obtain a compound of formula X;
step 11: reacting the compound of formula X under the action of another reducing agent to obtain pilocarpine,
wherein R₁, R₂ and R₃ are as defined above for the first aspect, and X is Cl, Br or I.

In one embodiment, the halogenating agent of step 8 is as described above in the fourth aspect.

In one embodiment, in step 9, the compound of formula I is subjected to Reformatsky condensation and lactonization reactions with 1-methylimidazole-5-carbaldehyde in the presence of zinc dust, dialkyl aluminum chloride, and cuprous bromide in an aprotic organic solvent to give the compound of formula IX. The aprotic organic solvent is as described above.

In one embodiment, the reducing agent described in step 10 is a reducing agent for catalytic hydrogenation or reduction catalysis, wherein the reducing agent for catalytic hydrogenation is selected from the group consisting of Ni, Pd/C, Pt/C, PtO₂/C with hydrogen, ammonium chloride and ammonium nitrate. The reducing agent for the reduction catalysis is selected from the group consisting of sodium borohydride, lithium borohydride, sodium cyanoborohydride, potassium borohydride, borane, aluminum oxide, lithium aluminum hydride.

In one embodiment, the another reducing agent described in step 11 is selected from any one of sodium borohydride, lithium borohydride, sodium cyanoborohydride, potassium borohydride, borane, red aluminum, lithium aluminum hydride, or any combination thereof.

In one embodiment, the process further comprises reacting pilocarpine with an acid to prepare a salt thereof, such as a hydrochloride or nitrate salt thereof.

In a preferred embodiment, the present invention provides a compound of formula II selected from the following compounds:

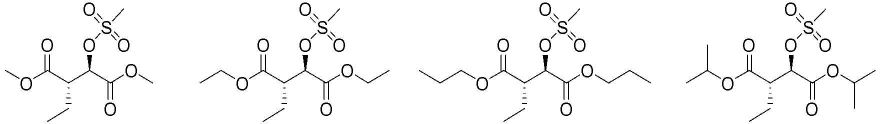

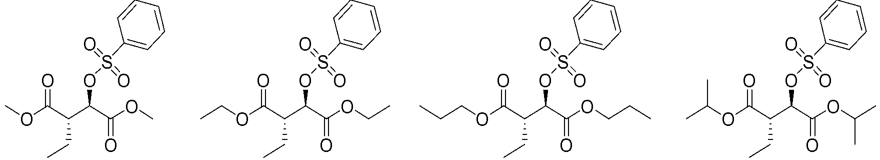

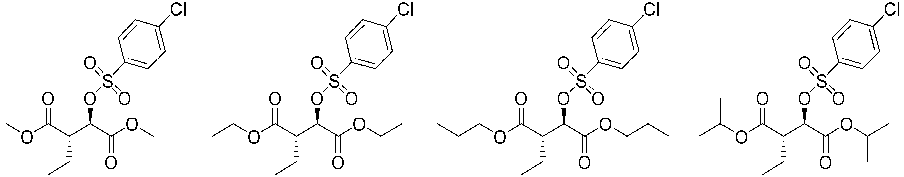

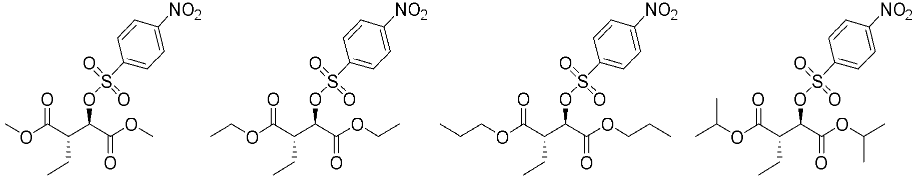

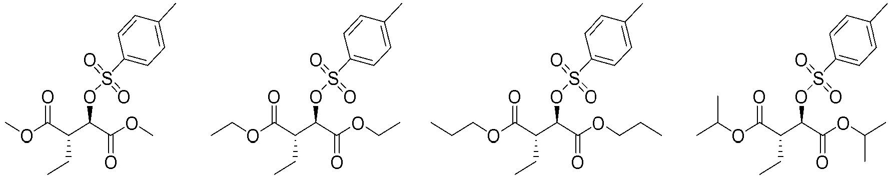

In a sixth aspect, the present invention provides a compound of formula I: 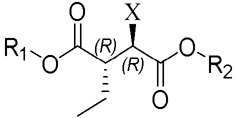
wherein R₁ and R₂ are as described above for the first aspect, and X is Cl, Br or I,
provided that the following are excluded: when X is Br, R₁ and R₂ are both Me, or R₁ is Me and R₂ is Et or t-butyl; or when X is I, R₁ is Me and R₂ is Et.

In one embodiment, the compounds of formula I do not include the following four compounds:

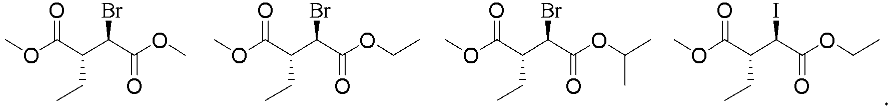

In the above process, the organic solvent to be used is not particularly limited as long as it can dissolve the starting material compound and does not participate in the reaction. Preferably, the organic solvent used may be selected from toluene, hexane, n-heptane, methyl acetate, ethyl acetate, isopropyl acetate, acetonitrile, tetrahydrofuran, isopropyl ether, methyl tert-butyl ether, dichloromethane, acetone, methanol, ethanol, isopropanol, or a mixture of two or more thereof.

In the above-mentioned process, the reaction temperature is not particularly limited either, and falls within the ordinary reaction temperature range.

In the above-mentioned process, the conventional quenching reaction means adding an aqueous solution, an ammonium chloride solution, an aqueous hydrochloric acid solution, an aqueous phosphoric acid solution or the like to the reaction solution to terminate the reaction or destroy the reaction system.

### Examples

The abbreviated terms used and other descriptions:
LiHMDS refers to lithium hexamethyldisilazide.
NaHMDS refers to sodium hexamethyldisilazide.
LDA refers to lithium diisopropylamide.
NCS refers to N-chlorosuccinimide.
NBS refers to N-bromosuccinimide.

The process of the present invention will be further illustrated by the following examples. It should be understood that the following examples are provided only for the purpose of enabling a better understanding of the present invention, and are not intended to limit the scope of the present invention in any way.

Unless otherwise indicated, the raw materials and reagents used in the examples of the present invention were obtained commercially or by known methods, purity and chiral values were measured by high performance liquid chromatography, and confirmation of the target product was confirmed by agreement with HPLC retention values of the standards.

### Example 1: preparation of compound of formula IV wherein R₁ and R₂ are methyl (Me)

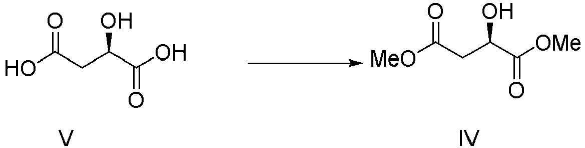

Under the protection of nitrogen, 100 g of L-malic acid and 200 g of methanol were added to a 500 mL flask, stirred to dissolve, and cooled to 0 to 10°C. 180 g of thionyl chloride was added dropwise, and stirred overnight at room temperature. After the reaction was completed, adding solid sodium bicarbonate to adjust the system to be neutral, concentrating to dryness under reduced pressure, adding 1000 mL of dichloromethane and 300 mL of water, standing and separating the layers, washing the organic layer twice with water (100 g × 2). The organic layer was concentrated to dryness to obtain 120g of oil with a yield of 99%.
**¹H NMR** (500 MHz, CDCl₃) δ 4.53 (dd, *J* = 6.1, 4.2 Hz, 1H), 3.80 (s, 3H), 3.61 (s, 3H), 2.92 - 2.88 (m, 1H), 2.83 - 2.75 (m, 1H).

### Example 2: preparation of compound of formula III wherein R₁ and R₂ are methyl (Me)

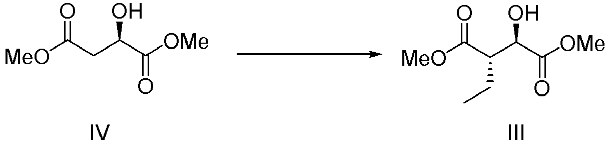

Adding 40g of the compound of formula IV prepared in Example 1 and 400mL of tetrahydrofuran into a 1000mL flask, stirring to dissolve, cooling to -70 to -80 °C, and adding 543mL of 1M LiHMDS in tetrahydrofuran dropwise. After the dropwise addition was completed, keeping the reaction at -70 to -80 °C and stirring for 1 hour continuously. Adding 29.6mL of ethyl iodide dropwise, and after the dropwise addition was completed, the reaction was allowed to warm up to 0-5 °C and reacted for 20 hours while keeping this temperature. Cooling the reaction to -70 to -80 °C, adding ammonium chloride solution for quenching, warming up to room temperature, adding hydrochloric acid to adjust the pH to 5-6, concentrating under reduced pressure, adding 500mL ×2 of ethyl acetate for extraction. The organic layer was dried over anhydrous Na₂SO₄, filtered and concentrated under reduced pressure to obtain 45g of oil with a yield of 96%.
**¹H NMR** (500 MHz, CDCl₃) δ 4.30 (d, *J* = 5.0 Hz, 1H), 3.72 (s, 3H), 3.63 (s, 3H), 2.82 - 2.77 (m, 1H), 1.90 - 1.85 (m, 1H), 1.75 - 1.70 (m, 1H), 1.01(t, *J* = 7.2 Hz, 3H).

### Example 3: preparation of compound of formula II wherein R₁ and R₂ are methyl (Me) and R₃ is p-nitrobenzenesulfonyl (Ns)

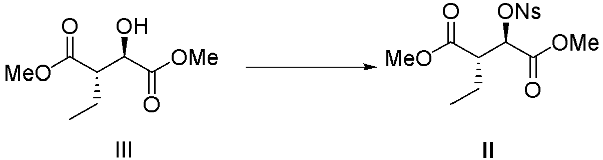

Dissolving 18g of the compound of formula III prepared in Example 2 in 200mL of dichloromethane, sequentially adding 14mL of Et₃N and 1g of DMAP, cooling in an ice bath, adding a solution of *p*-nitrobenzenesulfonyl chloride (NsCl) in DCM (22 g of NsCl +100mL of dichloromethane) dropwise, stirring at 0-5 °C for 2-3 hrs after the addition is finished, and then stirring at room temperature for 3hrs. Adding 1N HCl (50mL ×2) for washing, washing with water, drying over anhydrous Na₂SO₄, filtering, concentrating the filtrate to dryness, and adding ethyl acetate/hexane for crystallization. 29.1 g of off-white solid was obtained with a yield of 81.7% and a purity of 99%.
**¹H NMR** (500 MHz, DMSO) δ 8.49 - 8.30 (m, 2H), 8.18 - 8.04 (m, 2H), 5.16 (d, *J* = 6.2 Hz, 1H), 3.72 (s, 3H), 3.63 (s, 3H), 2.94 (dt, *J=* 8.1, 6.2 Hz, 1H), 1.82 - 1.71 (m, 1H), 1.60 - 1.51 (m, 1H), 0.96 (t, *J* = 7.4 Hz, 3H).

### Example 4: preparation of compound of formula I wherein R₁ and R₂ are methyl (Me) and X is bromo

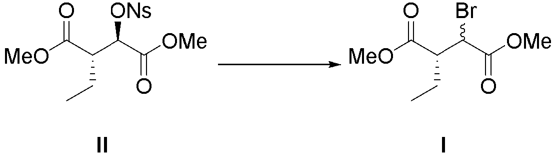

Dissolving 28.6 g of the raw material prepared according to the method of Example 3 in 335 mL of DMF, adding 33.3 g of lithium bromide, heating up to 40-45 °C, keeping the temperature and stirring for 6 hours. Adding 350 mL of water and 70 mL of methyl tert-butyl ether, standing and separating the layers, extracting the aqueous layer with 70 mL of methyl tert-butyl ether, combining organic layers, washing once with water, drying over anhydrous Na₂SO₄, filtering, and concentrating the filtrate to obtain 17.7 g of oil with a yield of 94% and a purity of 95%. **¹H NMR** (500 MHz, DMSO) δ 4.63 (dd, *J* = 24.1, 9.7 Hz, 1H), 3.73 (dd, *J* = 21.0, 6.3 Hz, 3H), 3.65 (d, *J* = 17.2 Hz, 3H), 3.03 - 2.93 (m, 1H), 1.93 - 1.71 (m, 1H), 1.65 - 1.46 (m, 1H), 0.86 (dt, *J=* 9.5, 7.5 Hz, 3H).

### Example 5: preparation of compound of formula IV wherein R₁ and R₂ are ethyl (Et)

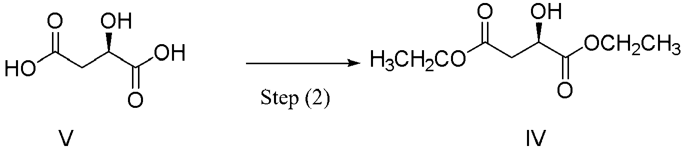

Under the protection of nitrogen, 100 g of L-malic acid and 200 g of ethanol were added to a 500 mL flask, stirred to dissolve, and cooled to 0 to 10°C. 180 g of thionyl chloride was added dropwise, and stirred overnight at room temperature. After the reaction was completed, adding solid sodium bicarbonate to adjust the system to be neutral, concentrating to dryness under reduced pressure, adding 1000 mL of dichloromethane and 300 mL of water, standing and separating the layers, washing the organic layer twice with water (100 g × 2). The organic layer was concentrated to dryness to obtain 140.3g of oil with a yield of 99%.

### Example 6: preparation of compound of formula III wherein R₁ and R₂ are ethyl (Et)

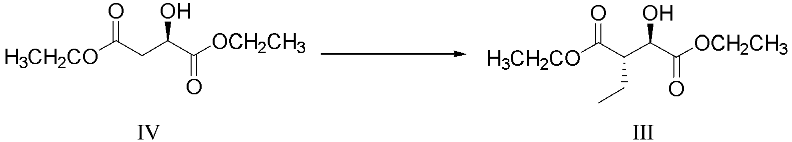

Adding 56.7g of the compound of formula IV prepared in Example 5 and 400mL of tetrahydrofuran into a 1000mL flask, stirring to dissolve, cooling to -70 to -80 °C and adding 543mL of 1M LiHMDS in tetrahydrofuran dropwise. After the dropwise addition was completed, keeping the reaction at -70 to -80 °C and stirring for 1 hour continuously. Adding 29.6mL of ethyl iodide dropwise, and after the dropwise addition was completed, the reaction was allowed to warm up to 0-5 °C and reacted for 20 hours while keeping this temperature. Cooling the reaction to -70 to -80 °C, adding ammonium chloride solution for quenching, warming up to room temperature, adding hydrochloric acid to adjust the pH to 5-6, concentrating under reduced pressure, adding 500mL ×2 of ethyl acetate for extraction. The organic layer was dried over anhydrous Na₂SO₄, filtered and concentrated under reduced pressure to obtain 62.5g of oil with a yield of 96%.

### Example 7: preparation of compound of formula II wherein R₁ and R₂ are Ethyl (Et) and R₃ is p-nitrobenzenesulfonyl

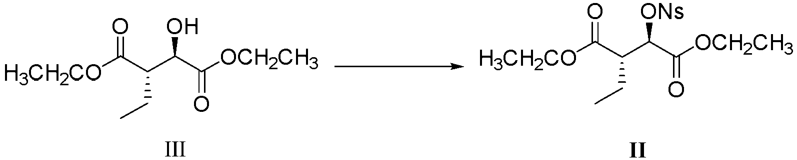

Dissolving 20.6g of the compound of formula III prepared in Example 6 in 250mL of dichloromethane, sequentially adding 14mL of Et₃N and 1g of DMAP, cooling in an ice bath, adding a solution of NsCl in DCM (22 g of NsCl +100mL of dichloromethane) dropwise. After the addition is finished, the reaction was stirred at 0-5 °C for 2-3 hrs and then stirred at room temperature for 3hrs. Adding 1N HCl (50mL ×2) for washing, washing with water, drying over anhydrous Na₂SO₄, filtering, concentrating the filtrate to dryness, and adding ethyl acetate/hexane for crystallization. 31.2 g of off-white solid was obtained with a yield of 81.9% and a purity of 99%.

### Example 8: preparation of compound of formula I wherein R₁ and R₂ are ethyl (CH₂CH₃) and X is bromo

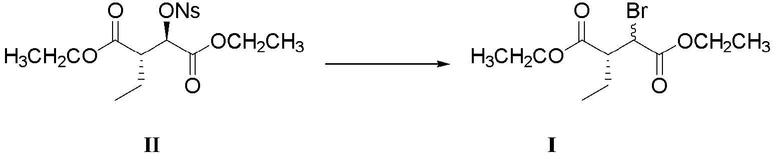

Dissolving 92.5 g of the compound prepared according to the method of Example 7 in 1000 mL of DMF, adding 100 g of lithium bromide, heating up to 40-45 °C, keeping the temperature and stirring for 6 hours. Adding 1000 mL of water and 200 mL of methyl tert-butyl ether, standing and separating the layers, extracting the aqueous layer with 200 mL of methyl tert-butyl ether, combining organic layers, washing once with water, drying over anhydrous Na₂SO₄, filtering, and concentrating the filtrate to obtain 58 g of oil with a yield of 90% and a purity of 96%.

### Example 9: preparation of compound of formula IV wherein R₁ and R₂ are isopropyl

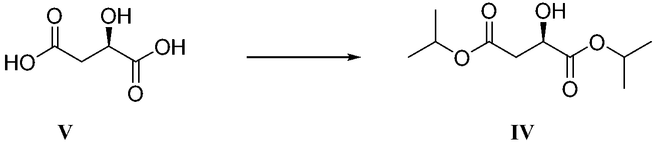

Under the protection of nitrogen, 100 g of L-malic acid and 200 g of isopropanol were added to a 500 mL flask, stirred to dissolve, and cooled to 0 to 10°C. 180 g of thionyl chloride was added dropwise, and stirred overnight at room temperature. After the reaction was completed, adding solid sodium bicarbonate to adjust the system to be neutral, concentrating to dryness under reduced pressure, adding 1000 mL of dichloromethane and 300 mL of water, standing and separating the layers, washing the organic layer twice with water (100 g × 2). The organic layer was concentrated to dryness to obtain 163g of oil with a yield of 100%.

In analogy to the above examples, a compound of formula II wherein R₁ and R₂ are isopropyl and R₃ is *p*-nitrobenzenesulfonyl was prepared, and then a compound of formula I wherein R₁ and R₂ are isopropyl and X is bromo.

### Example 10: preparation of pilocarpine hydrochloride

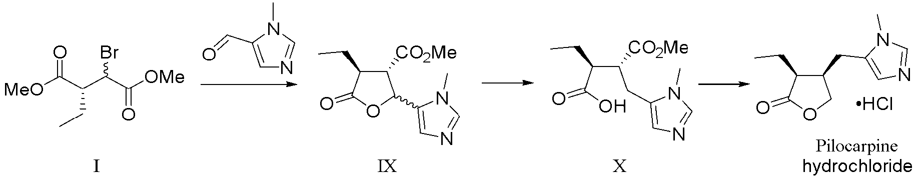

Under the protection of nitrogen, 32.1g of zinc powder, 6.5g of CuBr and 800mL of THF were added to a 1000 mL flask, and 430mL of 0.9M Me₂AlCl solution was added dropwise at room temperature. After the addition was completed, the reaction was stirred at room temperature for 20 min and then cooled to -5 to -10 °C, and 800mL of the solution of a compound of formula I wherein R₁ and R₂ are methyl and X is bromine (87.9 g) and 1-methylimidazole-5-carbaldehyde (35.9 g) in THF was added dropwise. After the addition was completed, the reaction was stirred at -10 to -8 °C for 2 hrs and then stirred at room temperature for 30 min, and then cooled to -5 to -10 °C. Dropwise adding 500mL of 50% MeOH aqueous solution, the reaction was stirred for 20min at room temperature, and then filtered with celite. The filter cake was washed successively with 1200mL of MeOH and 500mL of 10% hydrochloric acid/MeOH, the filtrate was evaporated to dryness under reduced pressure. The residue was added with 2500mL of 1.0M H₃PO₃ solution and extracted twice with methyl tert-butyl ether. The aqueous layer was added with ethyl acetate and neutralized with solid Na₂CO₃, and filtered with celite. The layers were separated and the aqueous layer was extracted twice with ethyl acetate. The extracts were combined, washed with saturated NaCl solution, dried over anhydrous Na₂SO₄, filtered, and the filtrate was evaporated to dryness to obtain 84.5 g of compound IX as an oil.

The oil obtained in the previous step was dissolved in 500mL of methanol, 10g of palladium/carbon was added, and a certain hydrogen pressure was maintained for reaction for 20 hours. The reaction mixture was filtered, and the filtrate was concentrated to obtain 84.7g of compound X as an oil.

The oil obtained in the previous step was dissolved in 2000mL of isopropanol, cooled to -5 to 0 °C, and 38g of lithium borohydride was added slowly in batches. After the addition was completed, the reaction was maintained at -5 to 0 °C and stirred for 1 hour. The reaction was then moved to room temperature for reaction for 20 hours. After the reaction was completed, hydrochloric acid was added to quench the system. The mixture was concentrated under reduced pressure, ammonia water was added until the system was alkaline. Dichloromethane (500mL ×2) was added for extraction, the organic layers were combined and concentrated under reduced pressure. The residue was taken up in ethanol, and hydrochloric acid was added dropwise until the system becomes acidic. The mixture was concentrated to dryness under reduced pressure, and ethanol and acetone were added for crystallization to obtain 60g of pilocarpus hydrochloride, wherein the overall yield is 70.8%, and the purity is 99.4%.

### Example 11: preparation of compound of formula VIII (wherein R₂ = Me):

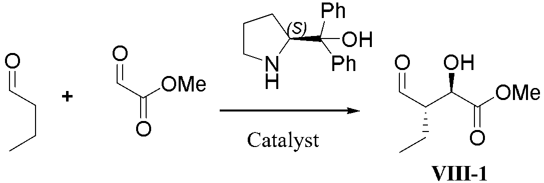

To a 5L reaction flask were added 2L of acetonitrile and 100mL of water, followed by 200 of n-butyraldehyde, 250g of methyl glyoxylate and 100g of catalyst, and the mixture was stirred at room temperature overnight. After TLC showed that the reaction is complete, acetonitrile was removed by rotary evaporation, 500 mL of methyl tert-butyl ether was added, and the mixture was stirred and separated. The aqueous layer was extracted with 500mL of methyl tert-butyl ether, the organic phases were combined, washed with saturated brine, dried over anhydrous sodium sulfate, filtered and rotary evaporated to dryness to obtain 444.5 g of the compound of formula VIII-1 as a light yellow oil (yield: 100%), which was used directly in the next reaction.

### Example 12: preparation of compound of formula VIII (wherein R₂ = isopropyl):

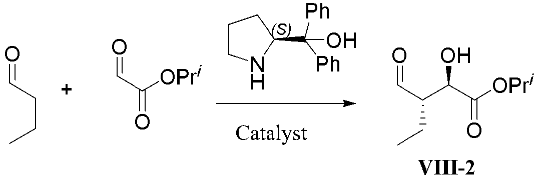

To a 5L reaction flask were added 2L of acetonitrile and 100mL of water, followed by 200 of n-butyraldehyde, 260g of isopropyl glyoxylate and 100g of catalyst, and the mixture was stirred at room temperature overnight. After TLC showed that the reaction is complete, acetonitrile was removed by rotary evaporation, 500 mL of methyl tert-butyl ether was added, and the mixture was stirred and separated. The aqueous layer was extracted with 500mL of methyl tert-butyl ether, the organic phases were combined, washed with saturated brine, dried over anhydrous sodium sulfate, filtered and rotary evaporated to dryness to obtain 522.1 g of light yellow oil in 100% yield, which was used directly in the next reaction.

### Example 13: preparation of compound of formula VII (wherein R₂ = Me):

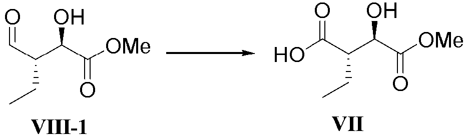

125.0 g of sodium dihydrogen phosphate aqueous solution was added to the product obtained in Example 11, 650g of 30% hydrogen peroxide and 750mL of 25% sodium chlorite aqueous solution were sequentially added dropwise while cooling in an ice bath. After the dropwise addition was completed, the mixture was stirred at room temperature for 1 to 2 hours. Sodium bisulfite aqueous solution was added dropwise under ice bath cooling until the potassium iodide test paper does not turn blue when used to test the aqueous solution. Acetonitrile was removed by concentration under reduced pressure, and after concentrating, liquid base was added dropwise under ice bath cooling until the pH value of the aqueous layer is 8-9. Extracting impurities twice with dichloromethane and the organic layer was discarded. Refined hydrochloric acid was added dropwise into the aqueous layer until the pH value of the aqueous layer is 1-2, and then the aqueous layer was extracted three times with dichloromethane. The organic phases were combined and concentrated under reduced pressure to obtain 452.3 g of the compound of formula VII with the yield of 92.5%.

### Example 14: preparation of compound of formula VI (wherein R₁ and R₂ are Me):

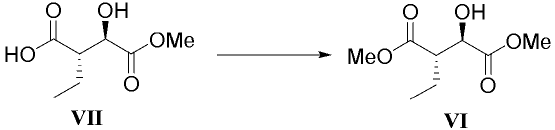

200.0 g of the compound of Example 13 were dissolved in 600 g of methanol, 60 g of concentrated sulfuric acid were added dropwise at room temperature, and the mixture was allowed to react overnight at 50 to 60 °C. TLC showed that the raw materials are completely reacted. The reaction system was allowed to cool to room temperature, 70g of triethylamine was added dropwise at room temperature, the mixture was distilled under reduced pressure, the residue was extracted twice with dichloromethane, the organic phases were combined, washed with saturated brine, dried over anhydrous sodium sulfate, filtered and rotary evaporated to dryness to obtain 216.0 g of the compound of formula VI. Yield: 100%.

### Example 15: preparation of compound of formula II (wherein R₁ and R₂ are Me, R₃ is tosyl)

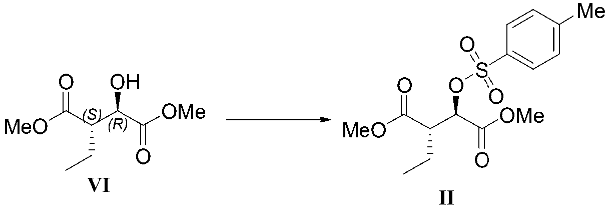

216.0g of the compound of Example 14 were dissolved in 1000 g of dichloromethane, 100g of triethylamine and 10g of DMAP were added, then, 700g of a dichloromethane solution containing 217.0g of tosyl chloride was added dropwise in an ice bath. After the dropwise addition, the reaction was carried out in ice bath for 3-4 hours. TLC showed that the raw materials are completely reacted. 500 mL of 1M aqueous hydrochloric acid solution was added dropwise, the mixture was stirred and separated, the aqueous layer was extracted with 200mL of dichloromethane, the organic phases were combined, washed with 500 mL of 20% aqueous sodium bicarbonate solution, then washed with 500 mL of water, concentrated under reduced pressure and then distilled with n-heptane for 2 times. The crude product was dissolved in 300mL ethyl acetate and heated up to 40.0 - 50.0 °C to obtain a clear solution. 1000mL of n-heptane were added dropwise at 40.0 - 50.0 °C, and after the dropwise addition, the mixture was cooled to room temperature and stirred for 3-4 hours at room temperature. The mixture was filtered, soaked and washed once with 200mL n-heptane, and the solid was dried to obtain 365.9 g of the compound of formula II (wherein R₁ and R₂ are Me, and R₃ is tosyl) with a yield of 93.6% and the HPLC purity of 98.5%.

The above-mentioned embodiments and examples further illustrate the purpose, technical solutions and advantages of the present invention in detail. It should be understood that the above-mentioned embodiments and examples are only illustrative of the present application and are not intended to limit the scope of the present application. Any modifications, equivalent substitutions, improvements, etc. made within the spirit and principles of the present invention shall be included in the protection scope of this application.

## Claims

1. A compound of formula II: 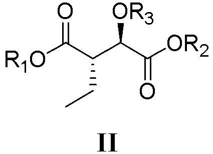
wherein R₃ is -S(O)₂-Rₓ and Rₓ is C₁₋₈alkyl, C₃₋₈cycloalkyl, C₆₋₁₀aryl, or C₆₋₁₀aryl-C₁₋₈alkyl, each of which is optionally substituted with one or more substituents selected from the group consisting of C₁₋₈alkyl, C₁₋₈alkoxy, haloC₁₋₈alkyl, halogen and nitro, and
R₁ and R₂ may be the same or different, and are each independently selected from the group consisting of C₁₋₈alkyl, C₃₋₈cycloalkyl, C₆₋₁₀aryl, or C₆₋₁₀aryl-C₁₋₈alkyl, wherein said C₁₋₈alkyl, C₃₋₈cycloalkyl, C₆₋₁₀aryl, or C₆₋₁₀aryl-C₁₋₈alkyl are each optionally substituted with one or more of C₁₋₈alkyl, C₁₋₈alkoxy, haloC₁₋₈alkyl, haloC₁₋₈alkoxy, halogen, hydroxy, and nitro,
preferably, R₁ and R₂ are each independently selected from the group consisting of C₁₋₈alkyl, benzyl, C₁₋₈alkyl substituted benzyl, C₁₋₈alkoxy substituted benzyl or halogen substituted benzyl.

2. The compound of claim 1, wherein R₃ is selected from the group consisting of methanesulfonyl, ethanesulfonyl, benzenesulfonyl, *p*-toluenesulfonyl, *p*-chlorobenzenesulfonyl, *p*-nitrobenzenesulfonyl, or *m*-dinitrobenzenesulfonyl.

3. A process for the preparation of the compound of formula II as defined in claim 1, comprising the steps of: 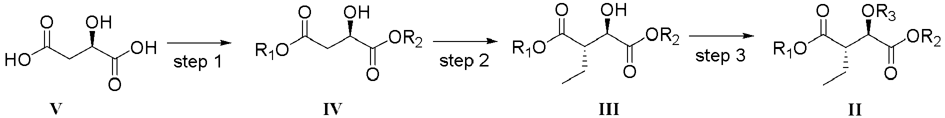 wherein R₁, R₂ and R₃ are as defined in claim 1 or 2,
step 1: reacting a compound of formula V with an alcohol of formula R_{y}-OH or with a sulfate ester to obtain a compound of formula IV, wherein R_{y} is as defined for R₁ and R₂ of claim 1;
step 2: reacting the compound of formula IV with a halogenated hydrocarbon CH₃CH₂X under basic conditions to obtain a compound of formula III, wherein X is Cl, Br or I; and
step 3: reacting the compound of formula III with a sulfonylating agent under basic conditions to obtain the compound of formula II.

4. The process according to claim 3, wherein the sulfate ester in step 1 is selected from the group consisting of dimethyl sulfate, diethyl sulfate, diisopropyl sulfate, or dipropyl sulfate.

5. The process according to claim 3, wherein the basic conditions of step 2 are selected from the group consisting of addition of one or more of LiHMDS, NaHMDS, LDA, n-butyllithium, t-butyllithium, NaH.

6. The process according to claim 3, wherein the basic conditions of step 3 are selected from the group consisting of addition of one or more of methylamine, ethylamine, propylamine, cyclopropylamine, n-butylamine, t-butylamine, n-pentylamine, isopentylamine, t-pentylamine, cyclopentylamine, hexylamine, cyclohexylamine, diethylamine, ethylenediamine, diisopropylethylamine, triethylamine, ethanolamine, aniline, phenethylamine and benzylamine.

7. The process according to claim 3, wherein the sulfonylating agent used in step 3 is X-S(O)₂-Rₓ, wherein Rₓ is as defined in claim 1 and X is Cl, Br or I, preferably said sulfonylating agent is selected from the group consisting of methanesulfonyl chloride, ethanesulfonyl chloride, benzenesulfonyl chloride, *p*-toluenesulfonyl chloride, *p*-chlorobenzenesulfonyl chloride, *p*-nitrobenzenesulfonyl chloride or *m*-dinitrobenzenesulfonyl chloride, methanesulfonyl bromide, ethanesulfonyl bromide, benzenesulfonyl bromide, *p*-toluenesulfonyl bromide, *p*-chlorobenzenesulfonyl bromide, *p*-nitrobenzenesulfonyl bromide or *m*-dinitrobenzenesulfonyl bromide.

8. A process for the preparation of the compound of formula II as defined in claim 1, comprising the steps of: 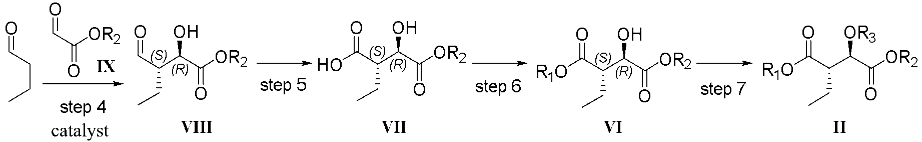 wherein R₁, R₂ and R₃ are as defined in claim 1 or 2,
step 4: carrying out condensation reaction of n-butyraldehyde and a compound of formula IX under catalysis to obtain a compound of formula VIII;
step 5: subjecting the compound of formula VIII to oxidation reaction to obtain a compound of formula VII;
step 6: esterifying the compound of formula VII with R₁-OH to obtain a compound of formula VI; and
step 7: subjecting the compound of formula VI to sulfonylation reaction to obtain the compound of formula II.

9. The process according to claim 8, wherein the catalyst used in step 4 is: 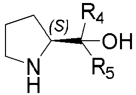 wherein R₄ and R₅ are each independently selected from the group consisting of C₆₋₁₀aryl, C₁₋₈alkyl substituted C₆₋₁₀aryl and halogen substituted C₆₋₁₀aryl, preferably phenyl.

10. The process according to claim 8, wherein the sulfonylating agent used in step 7 is X-S(O)₂-Rₓ, wherein Rₓ is as defined in claim 1 and X is Cl, Br or I, preferably said sulfonylating agent is selected from the group consisting of methanesulfonyl chloride, ethanesulfonyl chloride, benzenesulfonyl chloride, *p*-toluenesulfonyl chloride, *p*-chlorobenzenesulfonyl chloride, *p*-nitrobenzenesulfonyl chloride or *m*-dinitrobenzenesulfonyl chloride, methanesulfonyl bromide, ethanesulfonyl bromide, benzenesulfonyl bromide, *p*-toluenesulfonyl bromide, *p*-chlorobenzenesulfonyl bromide, *p*-nitrobenzenesulfonyl bromide or *m*-dinitrobenzenesulfonyl bromide.

11. A process for preparing a compound of formula I, 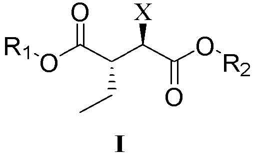 comprising: reacting a compound of formula II with a halogenating agent to obtain the compound of formula I 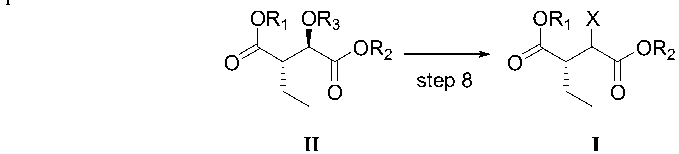 wherein R₁, R₂ and R₃ are as defined in claims 1 or 2, and X is Cl, Br or I.

12. The process according to claim 11, wherein said halogenating agent is selected from the group consisting of chlorine, NCS, trichloroisocyanuric acid, dichlorohydantoin, lithium chloride, sodium chloride, potassium chloride, tetrabutylammonium chloride, bromine, NBS, dibromohydantoin, tribromoisocyanuric acid, lithium bromide, sodium bromide, potassium bromide, tetrabutylammonium bromide, iodine, diiodohydantoin, lithium iodide, sodium iodide, potassium iodide and tetrabutylammonium iodide, or a combination of two or more of these halogenating agents.

13. A process for preparing pilocarpine, comprising the following steps: 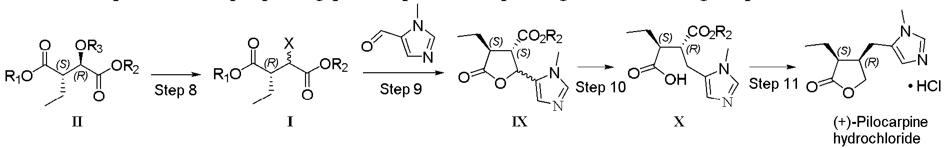
step 8: reacting a compound of formula II with a halogenating agent to obtain a compound of formula I;
step 9: reacting the compound of formula I with 1-methylimidazole-5-carbaldehyde to obtain a compound of formula IX;
step 10: reacting the compound of formula IX under the action of a reducing agent to obtain a compound of formula X;
step 11: reacting the compound of formula X under the action of another reducing agent to obtain pilocarpine,
wherein R₁ , R₂ and R₃ are as defined in claim 1, and X is Cl, Br or I.

14. The process according to claim 13, wherein said reducing agent in step 10 is a reducing agent for catalytic hydrogenation or reduction catalysis, wherein the reducing agent for catalytic hydrogenation is selected from the group consisting of Ni, Pd/C, Pt/C, PtO₂/C with hydrogen, ammonium chloride, formic acid, and ammonium formate, or a combination of two or more thereof, and the reducing agent for reduction catalysis is selected from the group consisting of sodium borohydride, lithium borohydride, sodium cyanoborohydride, potassium borohydride, borane, aluminum oxide, and lithium aluminum hydride, or a combination of two or more thereof.

15. The process according to claim 13, wherein said another reducing agent in step 11 is selected from the group consisting of sodium borohydride, sodium cyanoborohydride, potassium borohydride, borane, aluminum oxide, lithium aluminum hydride, or a combination of two or more thereof.

16. The process according to claim 13, further comprising reacting pilocarpine with an acid to produce a salt thereof, such as a hydrochloride or nitrate salt.

17. The process according to any one of claims 13-16, wherein said process further comprises steps 1-3: 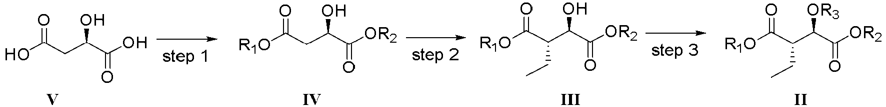 wherein steps 1-3 are as defined in any one of claims 3-7, and R₁, R₂ and R₃ are as defined in claim 1 or 2.

18. The process according to any one of claims 13-16, wherein said process further comprises steps 4-7: 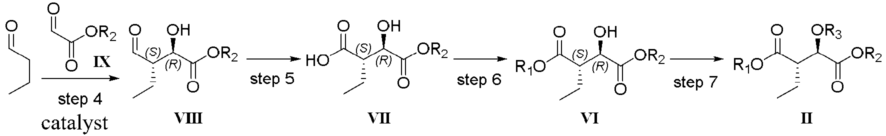 wherein steps 4-7 are as defined in any one of claims 8-10, and R₁, R₂ and R₃ are as defined in claim 1 or 2.

19. The compound of formula II according to claim 1, selected from the following compounds:

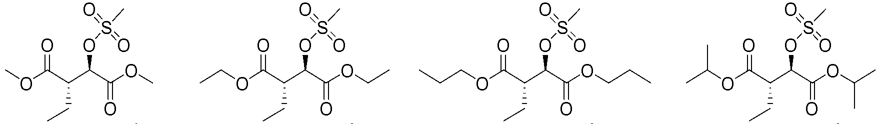

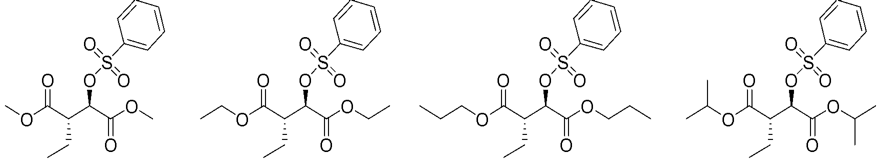

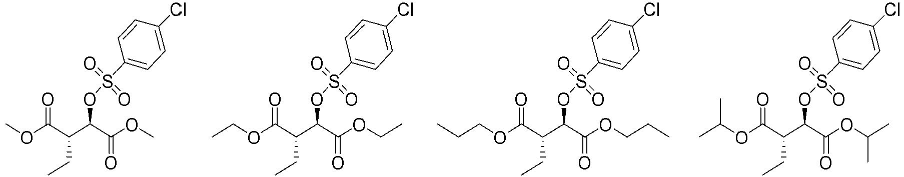

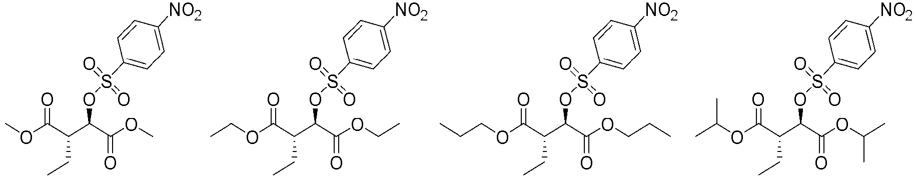

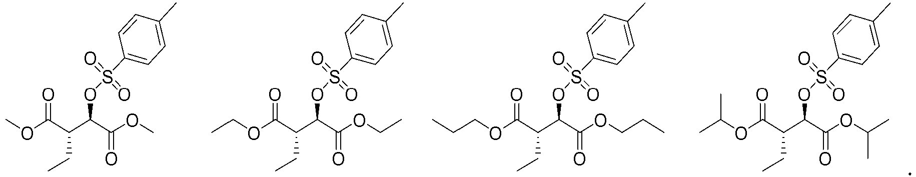

20. A compound of formula I: 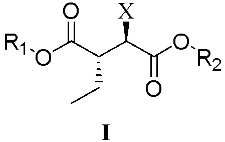
wherein R₁ and R₂ are as in claim 1, X is Cl, Br or I,
provided that the following are excluded: when X is Br, R₁ and R₂ are both Me, or R₁ is Me and R₂ is Et or t-butyl; or when X is I, R₁ is Me and R₂ is Et.
